# EUROPÄISCHE PATENTSCHRIFT

(11) **EP 0 528 876 B1**
(45) Veröffentlichungstag und Bekanntmachung des Hinweises auf die Patenterteilung: **12.04.1995**
(21) Anmeldenummer: 91909075.3
(22) Anmeldetag: 08.05.1991
(51) Int. Cl.: C07J 1/00

(54) **2-IOD-3-KETO-DELTA 4 -STEROIDE, VERFAHREN ZU IHRER HERSTELLUNG, SOWIE DEREN WEITERVERARBEITUNG**
2-IODO-3-KETO-DELTA 4 -STEROIDS, PROCESS FOR PRODUCING THEM AND THEIR FURTHER PROCESSING
STEROIDES 2-IODO-3-CETO-DELTA 4 , LEUR PROCEDE DE FABRICATION ET LEUR TRANSFORMATION ULTERIEURE

(30) Priorität: 09.05.1990 DE 4015247
(43) Veröffentlichungstag der Anmeldung: 03.03.1993
(73) Patentinhaber: SCHERING AKTIENGESELLSCHAFT, 13353 Berlin (DE)
(72) Erfinder: WESTERMANN, Jürgen, D-1000 Berlin 41 (DE); NICKISCH, Klaus, W-1000 Berlin 49 (DE); HARRE, Michael, D-1000 Berlin 41 (DE); ROHDE, Ralph, D-1000 Berlin 45 (DE)
(86) Internationale Anmeldenummer: DE9100386
(87) Internationale Veröffentlichungsnummer: WO9117175

(56) Entgegenhaltungen:
- EP-A- 0 290 378
- Steroids, Band 6, Nr. 6, Dezember 1965 H.J. Brodie et al.: "The catalytic reduction of 3beta-hydroxyandrost-5-en-17-one with tritium. The distribution and orientation of label in the product (1)" Seiten 659-674
- Journal of Medicinal Chemistry, Band 7, Nr. 4, Juli 1964 P.E. Shaw et al.: "Stroidal thiazenones and related compounds", Seiten 555-560
- Journal of Organic Chemistry, Band 28, Nr. 8, 12. August 1963 B. Berkoz et al.: "Steroids. CCXXI.1 Syntheses of some steroid dienes" Seiten 1976-1982

## Beschreibung

Die vorliegene Erfindung betrifft neue Zwischenprodukte der allgemeinen Formel I' 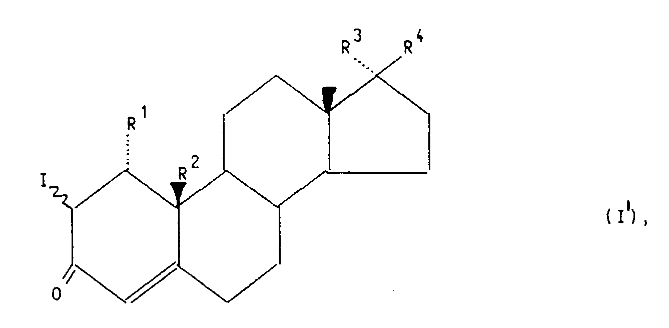
worin
- R¹: für ein Wasserstoffatom oder eine geradkettige oder verzweigte Alkylgruppe mit 1 bis 4 Kohlenstoffatomen,
- R²: für ein Wasserstoffatom oder eine Methylgruppe,
- R³: für ein Wasserstoffatom sowie
- R⁴: für eine Acyloxygruppe mit 1 bis 4 Kohlenstoffatomen im Acylrest oder aber
- R³ und R⁴: gemeinsam für ein Ketosauerstoffatom stehen, ausgenommen 2-Jod-androst-4-en-3,17-dion und 17β-Acetoxy-2-jod-androst-4-en-3-on.

Für R¹ steht vorzugsweise ein Wasserstoffatom oder eine Methylgruppe.
Als Acyloxygruppe R⁴ kommt in erster Linie die Acetoxygruppe in Betracht.
Insbesondere betrifft die vorliegende Erfindung nachstehende Verbindungen:
2β-Jod-1α-methylandrost-4-en-3,17-dion;
2α-Jod-1α-methylandrost-4-en-3,17-dion;
17β-Acetoxy-2-jod-1α-methyl-androst-4-en-3-on;
2-Iod-19-nor-androst-4-en-3,17-dion.

Die Zwischenprodukte der allgemeinen Formel I, das sind die Zwischenprodukte der allgemeinen Formel I' und die aus deren Umfang disclaimten 2-Jod-androst-4-en-3,17-dion und 17β-Acetoxy-2-jod-androst-4-en-3-on eignen sich in hervorragender Weise zur Einführung einer Δ¹-Doppelbindung in das Steroidgerüst bei gleichzeitigem Vorhandensein einer Δ⁴-Doppelbindung sowie gesättigter Carbonylgruppen.

Umsetzung einer Verbindung der allgemeinen Formel I mit einer Base in einem amidischen Lösungsmittel bei erhöhter Temperatur führt unter Abspaltung von Jodwasserstoff zu einer Verbindung der allgemeinen Formel II 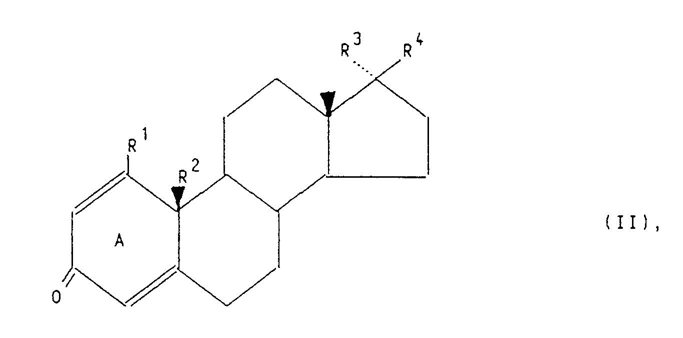
worin R¹, R², R³ und R⁴ die in Formel I angegebene Bedeutung haben.

Steht R² für ein Wasserstoffatom, so aromatisiert nach der Jodwasserstoffabspaltung der A-Ring unter Bildung einer Verbindung der allgemeinen Formel II' 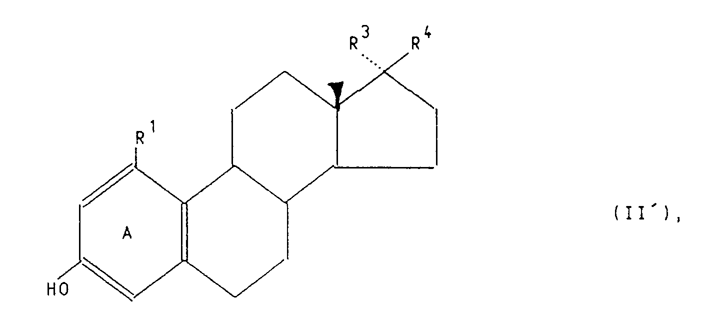
Die vorliegende Erfindung betrifft weiterhin Verfahren zur Herstellung der neuen Zwischenprodukte der allgemeinen Formel I.

Die aus dem Umfang der allgemeinen Formel I' disclaimten Verbindungen 2-Jod-androst-4-en-3,17-dion und 17β-Acetoxy-2α-jod-androst-4-en-3-on sind bereits bekannt. Erstere sowie deren 6,7-ditritiiertes Analogon gehen aus Steroids, Bd.6, Nr.6, 1965, S. 659-674 hervor, letztere ist schon in J. Med. Chem. Bd.7, Nr.4, 1964, S. 555-560 ernähnt und ihre Herstellung in J. Org. Chem., Bd.28, Nr.8, 1963, S. 1976-1982 beschrieben. Eine Verwendung als Zwischenprodukte findet sich dort nicht. Die zur Herstellung der bekannten Verbindungen beschriebenen Verfahren unterscheiden sich von dem hier offenbarten Verfahren, so daß sich der Gegenstand des anmeldegemäßen Verfahrens auch auf die im Stoffanspruch disclaimten Verbindungen erstreckt.

Beim Verfahren gemäß vorliegender Erfindung wird entweder eine Verbindung der allgemeinen Formel III 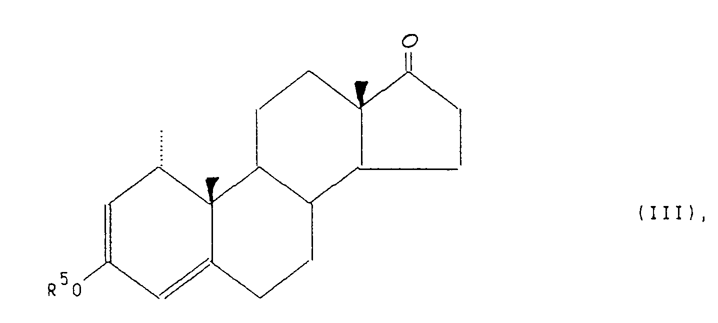
worin
R⁵ eine Acyl- oder Trialkylsilylgruppe mit bis zu 10 Kohlenstoffatomen in der Gruppe bedeutet (EP-A 0290378; DE-A 3 715 869), mit, gegebenenfalls in situ generiertem, N-Jodsuccinimid in einem Lösungsmittel, beispielsweise in einem Alkohol wie Methanol, Ethanol, Propanol oder dgl. zur Verbindung der allgemeinen Formel Ia 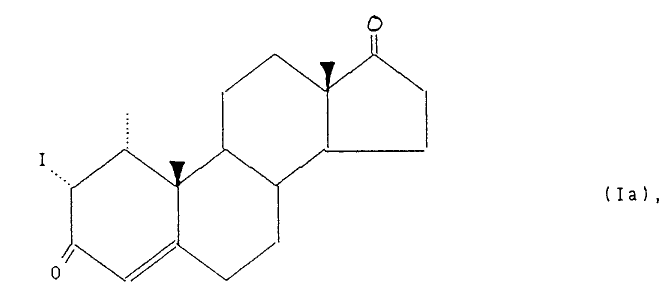
oder mit Jodmonochlorid oder -bromid in einem aprotischen Lösungsmittel in Gegenwart einer Base oder mit elementarem Jod in Essigsäure unter Zusatz einer Base zur Verbindung der Formel Ib 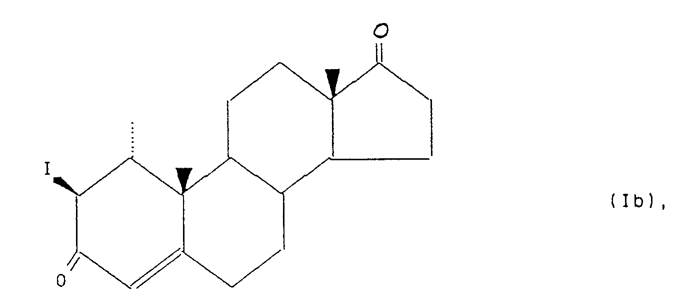
oder eine Verbindung der allgemeinen Formel IV 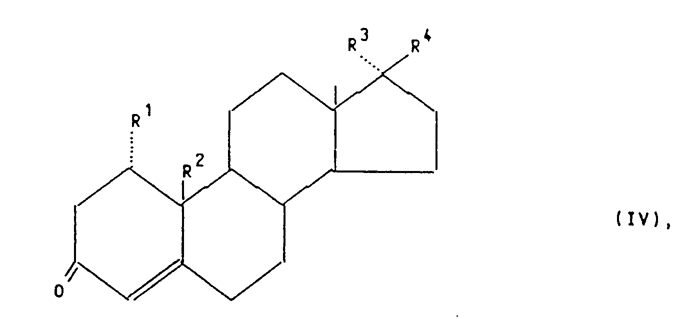
worin R¹, R², R³ und R⁴ die in Formel I angegebene Bedeutung haben, mit elementarem Jod, Jodmonochlorid oder -bromid, N-Jodsuccinimid oder einem N-Jodamid, wie 1,3-Dijodo-5,5-dimethylhydantoin oder einem anderen N-Jodimid in Gegenwart einer equimolaren Menge einer Kupferverbindung in Essigsäure oder in einem inerten Lösungsmittel, z.B. in einem Ether wie THF, unter Zusatz einer katalytischen Menge Essigsäure oder einer anderen C₁-C₃₋ Carbonsäure zu einer Verbindung der allgemeinen Formel Ic 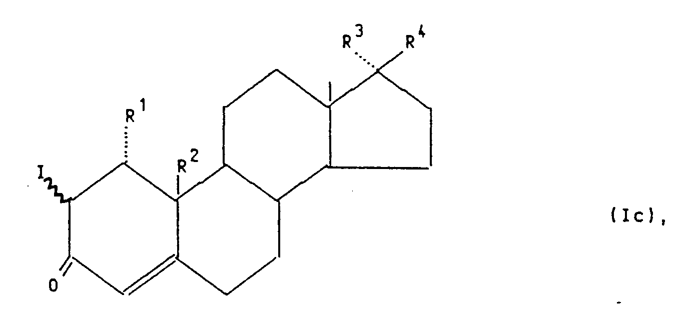
umgesetzt.

Durch Wahl der Reaktionsbedingungen läßt sich also die Stereochemie der Jodierungsreaktion steuern und zwar so, daß sich entweder die 2α-Form Ia, die 2β-Form Ib oder ein 2α⁷ 2β-Gemisch herstellen läßt. Regioselektive Jodierungen an 3,17-Diketosteroiden sind neu, ebenso die bereits erwähnte Δ¹-Einführung an 1-alkyl-substituierten Steroiden durch Jodwasserstoff-Abspaltung. Die bei Bromierungen häufig auftretende Reaktion in Allylposition (siehe die auf S. 6 angegebene Literatur: C. Djerassi; A. Corbellini) wird bei der Jodierung nicht beobachtet.

Ähnliche Verbindungen mit 2-Jod-3-keto-Δ⁴-Struktur (ohne 1-Alkylsubstituent) sind zwar bereits bekannt, sind aber in niedriger Ausbeute aus der entsprechenden Bromverbindung durch Halogenaustausch mit Natriumjodid hergestellt worden [J.Am.Chem.Soc. 72, 4077, (1962)].

Vorteilhafte Ausführungsformen des Verfahrens zur Herstellung von Verbindungen der allgemeinen Formel I ergeben sich aus den Unteranspruchen 4 bis 10. Die Umsetzung der Verbindungen der allgemeinen Formel III mit N-Jodsuccinimid wird vorzugsweise in einem Alkohol wie Methanol, Ethanol, Propanol oder 2-Propanol durchgeführt; die Umsetzung mit in situ generiertem N-Jodsuccinimid erfolgt am besten in Aceton oder Methanol.

Als Lösungsmittel für die Jodierung dar Verbindungen der allgemeinen Formel III bzw. IV mit Jodmonochlorid- oder bromid haben sich Ether wie Tetrahydrofuran oder Ketone wie beispielsweise Aceton oder Methylisobutylketon als besonders geeignet erwiesen.

Die Jodierung der Verbindungen der allgemeinen Formel III mit elementarem Jod wird in erster Linie in Eisessig als Lösungsmittel durchgeführt.

Als Base zur Jodierung mit Jodmonochlorid, -bromid oder mit elementarem Jod ist insbesondere an ein Carbonsäuresalz wie Natriumacetat oder ähnliche oder an ein Amin wie etwa Triethylamin oder Pyridin gedacht.

Kupfer-II-oxid, -acetat, -chlorid, -bromid, -jodid oder Kupfer-I-chlorid sind die bevorzugten Kupferverbindungen, in deren Gegenwart die Jodierung der Verbindungen der allgemeinen Formel IV vorgenommen wird.

Ein namhafter Vertreter einer Verbindung der allgemeinen Formel II ist das 1-Methyl-androsta-1,4-dien-3,17-dion (Atamestan); Atamestan ist ein sehr wirksamer Inhibitor der Estrogenbiosynthese (DE-A 33 22 285). Bisher wurde 1-Methyl-androsta-1,4-dien-3,17-dion durch Oxidation von 17β-Hydroxy-1-methyl-androsta-1,4-dien-3-on oder durch mikrobiologische Dehydrierung des 1-Methyl-5α-androst-1-en-3,17-dions (DE-A 35 12 328) hergestellt. Die Herstellung dieser beiden Verbindungen verläuft über mehrstufige Synthesen (5 Reaktionsschritte) und in geringen Ausbeuten.

Ein Problem stellt dabei die Einfuhrung der Δ¹-Doppelbindung an einem Steroid mit einer Δ⁴-Doppelbindung, insbesondere beim Vorhandensein einer 1α-Methylgruppe, dar.

Es ist kein geeignetes Verfahren bekannt, um aus einem 1α-Methyl-4-androsten-3,17-dion direkt zum 1-Methyl-androsta-1,4-dien-3,17-dion zu kommen.

Die Einführung einer Δ¹-Doppelbindung in ein 3-Keto-1-methyl-Δ⁴-steroid (z.B. in 1α-Methyltestosteron) mit 2,3-Dichlor-5,6-dicyano-benzochinon (DDQ) ergibt Δ^{1,4}- und Δ^{4,6}-Produkt im Verhältnis 1:2 und ist daher als wirtschaftliches Verfahren ungeeignet (A.B. Turner, H.J. Ringold, J. Chem. Soc. (C), 1967, 1720). In Synthesis 1981, 312 ist beschrieben, Verbindungen mit nur einer Ketogruppe in 2-Position zu jodieren. Die aufgeführten Beispiele lassen erkennen, daß gesättigte und ungesättigte Ketone (α,β-ungesättigte Carbonylverbindung) gleich schnell reagieren und sich in der Reaktivität nicht unterscheiden. Umso erstaunlicher ist es, daß die erfindungsgemäße Jodierung der 3,17-Diketone der allgemeinen Formel IV regioselektiv verläuft.
Die direkte Bromierung von z.B. 3-Keto-Δ⁴-Steroiden ist nicht geeignet, um 2-Brom-3-keto-Δ⁴-steroide herzustellen. So ergibt die Bromierung von Androst-4-en-3,17-dion als Produkt 2,6-Dibrom-androst-4-en-3,17-dion. Es erfolgt hierbei eine nicht selektive Bromierung in 2 und 6-Position am Steroid (C. Djerassi, J. Am. Chem. Soc., 72, 4534, (1950); A. Corbellini, Farmaco Ed. Sci., 19, 913 (1964)).

Ein einfacheres Verfahren zur Herstellung von 1-Methyl-androsta-1,4-dien-3,17-dion über einen kürzeren Syntheseweg geht aus der EP-A 0290 378 hervor. Androsta-1,4-dien-3,17-dion [J.Am.Chem.Soc., 79, 3920 (1957), Tetrahedron 4, 201 (1958)] wird durch Umsetzung mit einem Methylanionen liefernden Reagenz im Sinne einer Michael-Addition und durch anschließendes Abfangen des gebildeten Enolats in eine Verbindung der Formel III 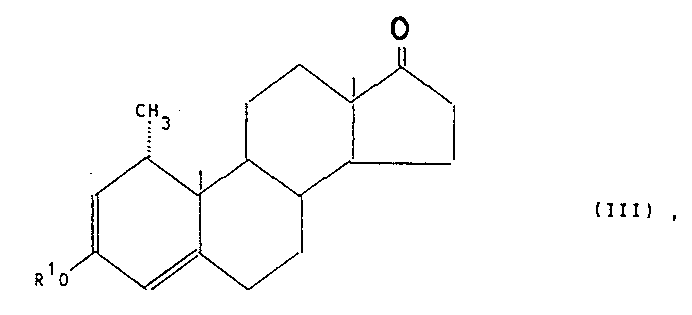
worin
R¹ einen Alkylrest mit 1-3 Kohlenstoffatomen oder eine Acyl- oder Trialkylsilylgruppe mit bis zu 10 Kohlenstoffatomen in der Gruppe bedeutet, überführt,
diese durch Bromierung in ein 2-Bromsteroid der Formel 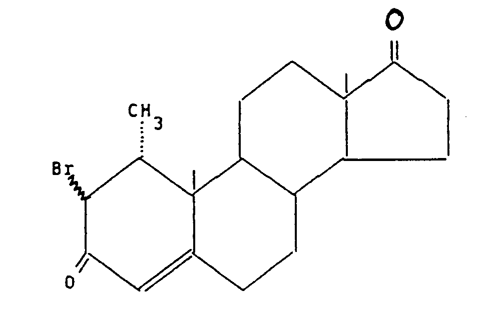
umgewandelt und das 2-Bromsteroid durch Dehydrobromierung zur Endverbindung weiterverarbeitet.
Die Jodierung einer Verbindung der Formel III gemäß vorliegender Erfindung verläuft sauberer und mit besserer Ausbeute als die in EP-A 0290 378 beschriebene Bromierung. Dies erlaubt eine einfachere Handhabung der Reaktion und Aufarbeitung der Jodide, z.B. durch Kristallisation.

Auch die Umsetzung einer Verbindung der allgemeinen Formel I mit einer Base zur Abspaltung von Jodwasserstoff verläuft sauberer als die Bromwasserstoffabspaltung. Überraschenderweise läßt sich bei Verwendung einer Jodverbindung der Formel I die Hauptnebenreaktion, nämlich die Bildung der Δ^{4,6}-Struktur, zurückdrängen (< 1%).

Dieses neue Verfahren ist aber nicht nur zur Herstellung von 1-Methylandrosta-1,4-dien-3,17-dion geeignet, sondern ist darüberhinaus allgemein nützlich zur Einfuhrung einer Δ¹-Doppelbindung am Steroid bei gleichzeitigem Vorhandensein einer Δ⁴-Doppelbindung und gesättigten Carbonylgruppen. Die vorliegende Erfindung betrifft daher auch ein solches Verfahren zur Einführung einer Δ¹-Doppelbindung.
Die Abspaltung von Jodwasserstoff aus einer Verbindung der allgemeinen Formel I gemäß voiliegender Erfindung wird vorzugsweise mit einer Base wie Magnesiumoxid, Lithium-, Natrium-, Kaliumcarbonat oder dgl. durchgeführt.

Als Lösungsmittel können vor allem basische Lösungsmittel wie Dimethylformamid oder N-Methylpyrrolidon Verwendung finden. Die Temperatur bei der Dehydrohalogenierung soll zwischen 100 und 150°C liegen.

Ist die Verbindung der allgemeinen Formel I ein 19-Nor-Steroid, d.h. R²=H, so kann das neue Verfahren zur Aromatisierung des A-Ringes des Steroidgerüstes herangezogen werden.

Eine alternative Methode zur Aromatisierung von Cyclohexenon-Systemen hat M.I. Al-Hassan kürzlich beschrieben (Synth. Commun. 19. (1989) 453; sowie weitere dort zitierte Methoden). Deprotonierung von 19-Nor-testosteron, Umsetzung mit problematischem Phenylselenylchlorid und Oxidation mit Persäure liefert nach Aufarbeitung β-Oestradiol in 40% Ausbeute.

Das neue Verfahren zur Einführung einer Δ¹-Doppelbindung liefert die entsprechenden Endprodukte in hoher Gesamtausbeute. Ausgehend von 1α-Methylandrost-4-en-3,17-dion sind nur 2 Reaktionsschritte erforderlich.

Die nachstehenden Ausführungsbeispiele dienen der näheren Erläuterung der Erfindung.

### Beispiel 1: 2β-jod-1α-methylandrost-4-en-3,17-dion

17.1 g (50 mmol) 3-Acetoxy-1α-methyl-androsta-2,4-dien-17-on (DE-A 3715869) werden in 235 ml Aceton gelöst und mit 8.2 g (100 mmol) wasserfreiem Natriumacetat versetzt. Nach Abkühlen auf -10°C werden unter Stickstoffatmosphäre 9.75 g (60 mmol) Chlorjod zugegeben. Es wird 30 Minuten bei -10°C nachgerührt. Die Reaktionslösung wird unter Rühren in 1 l mit 2 g Natriumthiosulfat versetztes Eiswasser gegeben. Der Feststoff wird abgesaugt und mit Wasser nachgewaschen. Nach Trocknen der Substanz werden 21 g der Titelverbindung (99 % der Theorie) vom Schmelzpunkt 119 °C erhalten.

### Beispiel 2: 1-Methylandrosta-1,4-dien-3,17-dion

Zu einer auf 130°C vorgeheizten Suspension von 0.73 g (10 mmol) Lithiumcarbonat in 10 ml Dimethylformamid werden 2.13 g (5 mmol) 2β-Jod-1α-methylandrost-4-en-3,17-dion aus Beispiel 1 eingetragen und 1.5 Stunden bei dieser Temperatur gerührt. Nach Abkühlen wird die Reaktionslösung auf 30 ml Wasser gegeben, mit Ethylacetat extrahiert und nach Trocknen über Natriumsulfat eingeengt. Das Rohprodukt wird an Kieselgel mit Ethylacetat/Hexan als Laufmittel chromatographiert. Nach Umkristallisation aus Ethylacetat werden 1.1 g 1-Methylandrosta-1,4-dien-3,17-dion (74 % der Theorie) vom Schmelzpunkt 169°C erhalten.

### Beispiel 3: 2α-Jod-1α-methylandrost-4-en-3,17-dion

3.42 g (10 mmol) 3-Acetoxy-1α-methyl-androsta-2,4-dien-17-on (DE-A 3715869) werden in 40 ml absolutem Methanol gelöst. Unter Stickstoffatmosphäre werden 2.25 g (10 mmol) N-Jodsuccinimid zugegeben und es wird 1 Stunde bei Raumtemperatur gerührt. Das Reaktionsgemisch wird unter Rühren in 70 ml Eiswasser gegeben und das Produkt mit Ethylacetat extrahiert. Die organische Phase wird mit 50 ml Wasser gewaschen und über Natriumsulfat getrocknet. Nach Chromatographie des Rohproduktes an Kieselgel mit Ethylacetat/Hexan als Laufmittel werden 2.36 g (55.4 % der Theorie) 2α-Jod-1α-methylandrost-4-en-3,17-dion als Feststoff erhalten.

### Beispiel 4: 1-Methylandrosta-1,4-dien-3,17-dion

147.78 mg (2 mmol) Lithiumcarbonat werden in 3 ml N-Methylpyrrolidon unter Stickstoffatmosphäre auf 130°C erhitzt. Zu dieser vorgeheizten Lösung werden 426 mg (1 mmol) 2α-Jod-1α-methyl-androst-4-en-3,17-dion aus Beispiel 3 gegeben. Die Lösung wird noch 2 h bei dieser Temperatur gerührt. Nach Abkühlen auf Raumtemperatur wird die Lösung in 20 ml Eiswasser gegegeben und das Produkt mit Ethylacetat extrahiert. Nach Abdampfen des Lösungsmittels wird das Rohprodukt mit Ethylacetat/Hexan als Laufmittel durch Chromatographie an kieselgel aufgereinigt. Einengen der Fraktionen und Umkristallisation aus Ethylacetat liefert 178 mg (60 % der Theorie) 1-Methylandrosta-1,4-dien-3,17-dion vom Schmelzpunkt 169 °C.

### Beispiel 5: 2β-Jod-1α-methylandrost-4-en-3,17-dion

68.95 g 88.8 %iges (0.179 mol) 3-Acetoxy-1α-methyl-androsta-2,4-dien-17-on (DE-A 3715869) werden in 800 ml Aceton gelöst, mit 32.9 g (0.4 mol) wasserfreiem Natriumacetat versetzt und unter Rühren auf -10°C abgekühlt. Anschließend werden unter Stickstoffatmosphäre 29 g (0.179 mol) Chlorjod (Jodmonochlorid) bei -10°C zugegeben und es wird noch 30 Minuten nachgerührt. Dann wird auf 6 l mit 6.65 g Natriumthiosulfat versetztes Eiswasser gegeben, das Produkt mit Essigester extrahiert, die organische Phase über Natriumsulfat getrocknet, filtriert und bis auf 200 ml eingeengt. Nach Stehenlassen über Nacht wird der ausgefallene Feststoff abgesaugt und getrocknet. Es werden 49 g (64% der Theorie) Produkt erhalten. Nach Chromatographie der Mutterlauge an Kieselgel mit Ethylacetat/Hexan als Laufmittel werden weitere 10 g (13 % der Theorie) der Titelverbindung erhalten. Die Gesamtausbeute beträgt 59 g (77 % der Theorie) an 2β-Jod-1α-methylandrost-4-en-3,17-dion.

### Beispiel 6: 1-Methylandrosta-1,4-dien-3,17-dion

19.40 g (0.262 mol) Lithiumcarbonat werden in 280 ml absolutem Dimethylformamid suspendiert und unter Stickstoffatmosphäre auf 130 °C erhitzt. Bei dieser Temperatur werden 56 g (0.131 mol) 2β-Jod-1α-methylandrost-4-en-3,17-dion aus Beispiel 5 portionsweise zugegeben. Unter Stickstoffatmosphäre wird die Lösung noch 1 h bei dieser Temperatur gerührt. Anschließend wird unter vermindertem Druck Dimethylformamid am Rotationsverdampfer abdestilliert. Der Rückstand wird in 400 ml Ethylacetat aufgenommen, mit 400 ml Wasser versetzt, die Wasserphase nach Phasentrennung mit 2 × 200 ml Ethylacetat nachextrahiert und die Ethylacetatphase mit 150 ml 10%iger Natriumthiosulfatlösung gewaschen und über Natriumsulfat getrocknet. Aus der auf 80 ml eingeengten Ethylacetatlösung werden noch Abkühlen 23.5 g Kristalle erhalten. Die Mutterlauge wird mit Hexan/Ethylacetat als Laufmittel an Kieselgel chromatographiert. Das chromatographisch aufgereinigte Produkt wird mit dem Kristallisat zusammengefaßt und aus Ethylacetat umkristallisiert. Nach Abfiltrieren und Trocknen werden 31.3 g 1-Methylandrosta-1,4-dien-3,17-dion (80.2 % der Theorie) als farblose Kristalle vom Schmelzpunkt 170 °C erhalten. Die Reinheit der so erhaltenen Substanz beträgt nach HPLC 99.85 %.

### Beispiel 7: 2-Jod-1α-methyl-androst-4-en-3,17-dion

Zu 10 g (33 mmol) 1α-Methyl-androst-4-en-3,17-dion und 7.33 g (40 mmol) CuO in 300 ml Eisessig werden unter Rühren bei Raumtemperatur 10.3 g (40 mmol) Jod gegeben. Unter Stickstoffatmosphäre wird 24 h bei 60 °C gerührt. Unter reduziertem Druck wird Essigsäure abdestilliert. Der Rückstand wird in 600 ml Wasser gegeben, das Produkt 3 mal mit je 200 ml Ethylacetat extrahiert, die vereinigten Ethylacetatphasen werden mit Natriumthiosulfatlösung, anschließend mit gesättigter Natriumcarbonatlösung neutral gewaschen und über Natriumsulfat getrocknet. Nach Einengen der Lösung und Trocknen der Substanz bei 0.8 Torr werden 15.0 g (106 % der Theorie) 2-Jod-1α-methyl-androst-4-en-3,17-dion als Rohprodukt erhalten.

### Beispiel 8: 1-Methyl-androsta-1,4-dien-3,17-dion

14.9 g 2-Jod-1α-methyl-androst-4-en-3,17-dion Rohprodukt aus Beispiel 7 und 4.73 g (64 mmol) wasserfreies Lithiumcarbonat werden in 95 ml N-Methylpyrrolidon 1 Stunde bei 130°C unter Stickstoffatmosphäre gerührt. Nach Abkühlen der Reaktionslösung wird auf 0.6 l Wasser gegeben und das Produkt 3 mal mit 450 ml Ethylacetat extrahiert. Die vereinigten Ethylacetatphasen werden über Natriumsulfat getrocknet und anschließend eingeengt. Der Rückstand wird an Kieselgel mit Ethylacetat/Hexan als Laufmittel chromatographiert. Nach Einengen der Fraktionen und Umkristallisation der Substanz aus Ethylacetat werden 6.9 g 1-Methyl-androsta-1,4-dien-3,17-dion (69 % der Theorie über 2 Stufen bezogen auf 1α-Methyl-androst-4-en-3,17-dion in Beispiel 7) vom Schmelzpunkt 168-169°C erhalten.

### Beispiel 9: 2-Jod-androst-4-en-3,17-dion

Zu 2.86 g (10 mmol) Androst-4-en-3,17-dion und 0.875 g (11 mmol) CuO in 30 ml Eisessig werden unter Rühren bei Raumtemperatur 2.79 g (11 mmol) Jod gegeben. Das Reaktionsgemisch wird unter Stickstoffatmosphäre 24 h bei 60 °C gerührt. Unter reduziertem Druck wird Essigsäure abdestilliert und der Rückstand mit 50 ml Wasser versetzt. Die Wasserphase wird 3 mal mit je 50 ml Ethylacetat extrahiert, die vereinigten Essigesterphasen werden mit Natriumthiosulfatlösung gewaschen und über Natriumsulfat getrocknet. Nach Einengen der Lösung werden 4.5 g Rohprodukt erhalten. Nach Umkristallisation aus Ethylacetat werden 2.9 g 2-Jod-androst-4-en-3,17-dion (70 % der Theorie) vom Schmelzpunkt 92°C erhalten.

### Beispiel 10: Androsta-1,4-dien-3,17-dion

412 mg (1 mmol) 2-Jod-androst-4-en-3,17-dion aus Beispiel 9 und 148 mg (2 mmol) wasserfreies Lithiumcarbonat werden in 2 ml Dimethylformamid 1 Stunde bei 120°C unter Stickstoffatmosphäre gerührt. Nach Abkühlen wird auf Wasser gegeben und mit Ethylacetat extrahiert. Die Ethylacetatlösung wird über Natriumsulfat getrocknet und anschließend eingeengt. Der Rückstand wird an Kieselgel mit Ethylacetat/Hexan als Laufmittel chromatographiert. Nach Einengen der Fraktionen werden 227 mg (80% der Theorie) Androsta-1,4-dien-3,17-dion vom Schmelzpunkt 138-139°C erhalten.

### Beispiel 11: 17β-Acetoxy-2-jod-1α-methyl-androst-4-en-3-on

6.88 g (20 mmol) 17β-Acetoxy-1α-methyl-androst-4-en-3-on und 1.75 g (22 mmol) CuO in 60 ml Essigsäure werden unter Rühren bei Raumtemperatur mit 5.58 g (22 mmol) Jod versetzt und unter Stickstoffatmosphäre 24 h bei 60 °C gerührt. Unter reduziertem Druck wird die Essigsäure weitgehend abdestilliert und der Rückstand in 100 ml Wasser aufgenommen. Die Wasserphase wird mit 3 mal mit je 100 ml Ethylacetat extrahiert. Die Essigesterphasen werden mit Natriumthiosulfatlösung gewaschen und über Natriumsulfat getrocknet. Nach Einengen der Lösung werden 10 g Rohprodukt erhalten. Umkristallisation aus Ethylacetat ergibt 8.8 g 17β-Acetoxy-2-jod-1α-methyl-androst-4-en-3-on (93 % der Theorie) vom Schmelzpunkt 102 °C.

### Beispiel 12: 17β-Acetoxy-1-methyl-androsta-1,4-dien-3-on

1.4 g (3 mmol) 17β-Acetoxy-2-jod-1α-methyl-androst-4-en-3-on aus Beispiel 11 und 0.443 g (6 mmol) wasserfreies Lithiumcarbonat werden in 6 ml Dimethylformamid 1 Stunde bei 120°C unter Stickstoffatmosphäre gerührt. Nach Abkühlen wird auf Wasser gegeben und mit Ethylacetat extrahiert. Die Ethylacetatlösung wird über Natriumsulfat getrocknet und anschließend eingeengt. Der Rückstand wird an Kieselgel mit Ethylacetat/Hexan als Laufmittel chromatographiert. Nach Einengen der Fraktionen werden 0.95 g (92 % der Theorie) 17β-Acetoxy-1-methyl-androsta-1,4-dien-3-on vom Schmelzpunkt 173°C erhalten.

### Beispiel 13: 2-Jod-19-nor-androst-4-en-3,17-dion

Zu 2.72 g (10 mmol) 19-Nor-androst-4-en-3,17-dion und 0.875 g (11 mmol) CuO in 30 ml Eisessig werden unter Rühren bei Raumtemperatur 2.79 g (11 mmol) Jod gegeben. Die Reaktion wird unter Stickstoffatmosphäre 24 h bei 60 °C gerührt. Unter reduziertem Druck wird Essigsäure abdestilliert und der Rückstand mit 50 ml Wasser versetzt. Die Wasserphase wird mit 3 mal 50 ml Ethylacetat extrahiert, die vereinigten Essigesterphasen werden mit Natriumthiosulfatlösung gewaschen und über Natriumsulfat getrocknet. Nach Einengen der Lösung werden 6.0 g Rohprodukt erhalten. Chromatographie des Rohproduktes an Kieselgel mit Ethylacetat/Hexan als Laufmittel und Umkristallisation aus Ethylacetat ergibt 2.9 g 2-Jod-19-nor-androst-4-en-3,17-dion (70 % der Theorie) vom Schmelzpunkt 108 °C.

### Beispiel 14: Oestron (1,3,5-Oestratrien-3-ol-17-on)

398 mg (1 mmol) 2-Jod-19-nor-androst-4-en-3,17-dion aus Beispiel 13 und 148 mg (2 mmol) wasserfreies Lithiumcarbonat werden in 2 ml Dimethylformamid 2 Stunden bei 120°C unter Stickstoffatmosphäre gerührt. Nach Abkühlen wird auf Wasser gegeben, mit 2 n HCl auf pH 3 angesäuert und das Produkt mit Ethylacetat extrahiert. Die Ethylacetatlösung wird über Natriumsulfat getrocknet und anschließend eingeengt. Der Rückstand wird an Kieselgel mit Ethylacetat/Hexan als Laufmittel chromatographiert. Nach Einengen der Fraktionen und Umkristallisation aus Ethylacetat werden 207 mg Oestron (1,3,5-Oestratrien-3-ol-17-on) (76 % der Theorie) vom Schmeizpunkt 258-260°C erhalten.

### Beispiel 15: 17β-Acetoxy-2-jod-androst-4-en-3-on

Zu 3.28 g (10 mmol) 17-Acetoxy-androst-4-en-3-on und 0.875 g (11 mmol) CuO in 30 ml Eisessig werden unter Rühren bei Raumtemperatur 2.79 g (11 mmol) Jod gegeben. Die Reaktionslösung wird unter Stickstoffatmosphäre 24 h bei 60 °C gerührt. Unter reduziertem Druck wird Essigsäure abdestilliert und der Rückstand in 50 ml Wasser aufgenommen. Die Wasserphase wird 3 mal mit je 50 ml Ethylacetat extrahiert, die Essigesterphasen mit Natriumthiosulfatlösung gewaschen und über Natriumsulfat getrocknet. Einengen der Lösung liefert 4.5 g Rohprodukt. Nach Umkristallisation aus Ethylacetat werden 2.85 g 17β-Acetoxy-2-jod-androst-4-en-3-on (65 % der Theorie) vom Schmelzpunkt 92°C erhalten.

### Beispiel 16: 17β-Acetoxy-androsta-1,4-dien-3-on

440 mg (1 mmol) 17β-Acetoxy-2-jod-androst-4-en-3-on aus Beispiel 15 und 148 mg (2 mmol) wasserfreies Lithiumcarbonat werden in 2 ml Dimethylformamid 1 Stunde bei 130°C unter Stickstoffatmosphäre gerührt. Nach Abkühlen wird auf Wasser gegeben und mit Ethylacetat extrahiert. Die Ethylacetatlösung wird über Natriumsulfat getrocknet und anschließend eingeengt. Der Rückstand wird an Kieselgel mit Ethylacetat/Hexan als Laufmittel chromatographiert. Nach Einengen der Fraktionen werden 0.3 g (91 % der Theorie) 17β-Acetoxy-androsta-1,4-dien-3-dion vom Schmelzpunkt 165 °C erhalten.

## Patentansprüche

1. Zwischenprodukte der allgemeinen Formel I' 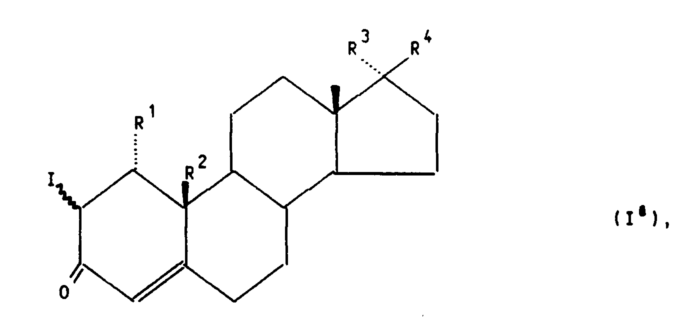 worin
R¹ für ein Wasserstoffatom oder eine geradkettige oder verzweigte Alkylgrinppe mit 1 bis 4 Kohlenstoffatomon,
R² für ein Wasserstoffatom oder eine Methylgruppe,
R³ für ein Wasserstoffatom sowie
R⁴ für eine Acyloxygruppe mit 1 bis 4 Kohlenstoffatomen im Acylrest odor aber
R³ und R⁴ gemeinsam für ein Ketosauerstoffatom stehen, ausgenommen 2-Jod-androst-4-en-3,17-dion und 17β-Acetoxy-2-jod-androst-4-en-3-on.

2. 2β-Jod-1α-methylandrost-4-en-3,17-dion;
2α-Jod-1α-methylandrost-4-en-3,17-dion;
17β-Acetoxy-2-jod-1α-methyl-androst-4-en-3-on;
2-Jod-19-nor-androst-4-en-3,17-dion;

3. Verfahren zur Herstellung von Zwischenprodukten der allgemeinen Formel I 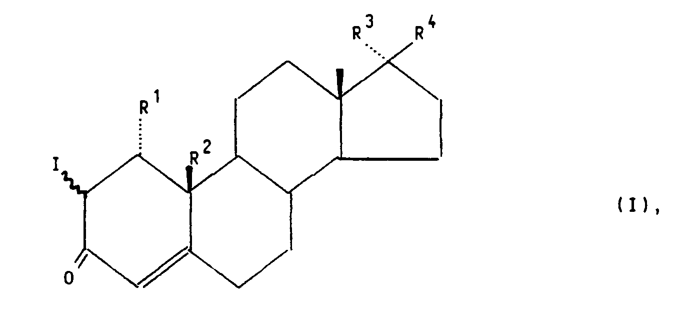 worin R¹, R², R³ und R⁴ die in Anspruch 1 angegebene Bedeutung haben, dadurch gekennzeichnet, daß entweder eine Verbindung der allgemeinen Formel III 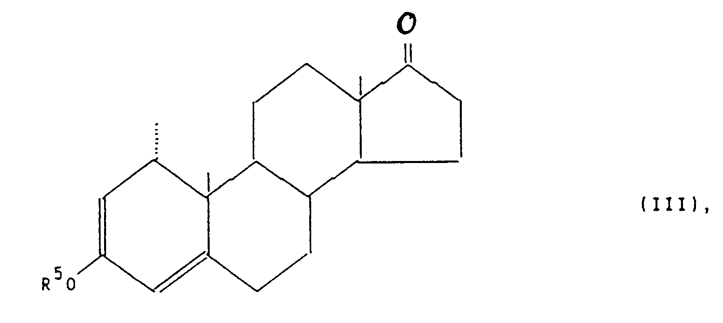 worin
R⁵ eine Acyl- oder Trialkylsilylgruppe mit bis zu 10 Kohlenstoffatomen in der Gruppe bedeutet, mit, gegebenenfalls in situ generiertem, N-Jodsuccinimid in einem Lösungsmittel zur Verbindung der Formel Ia 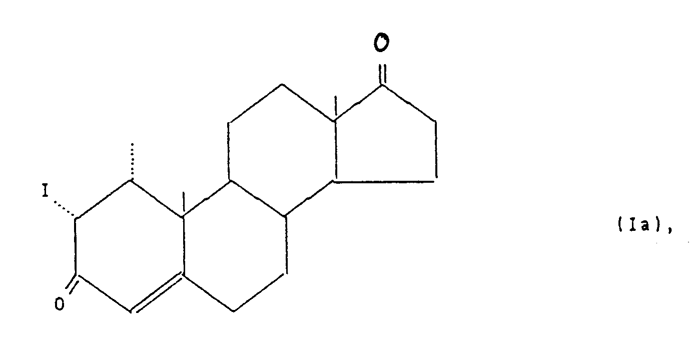 oder mit Jodmonochlorid oder -bromid in einem aprotischen Lösungsmittel in Gegenwart einer Base oder mit elementarem Jod unter Zusatz einer Base zur Verbindung der Formel Ib 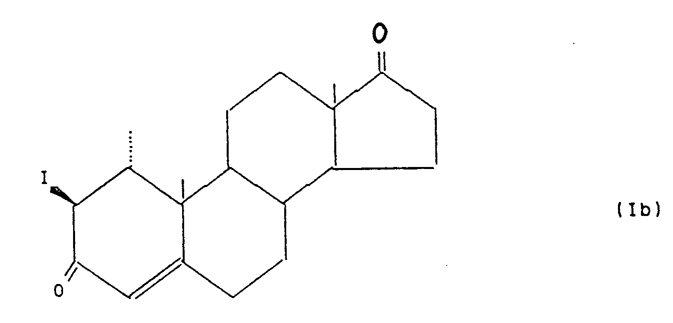 oder eine Verbindung der allgemeinen Formel IV 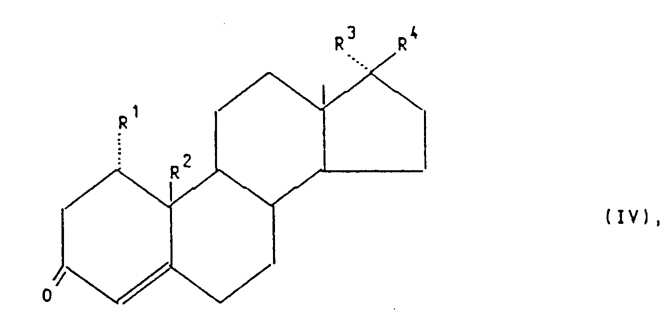 worin R¹, R², R³ und R⁴ die in Formel I angegebene Bedeutung haben, mit elementarem Jod, Jodmonochlorid oder -bromid, N-Jodsuccinimid oder einem anderen N-Jodamid oder einem anderen N-Jodimid in Gegenwart einer equimolaren Menge einer Kupferverbindung in Essigsäure oder in einem inerten Lösungsmittel unter Zusatz einer katalytischen Menge Essigsäure oder einer anderen C₁-C₃-Carbonsäure zu einer Verbindung der allgemeinen Formel Ic 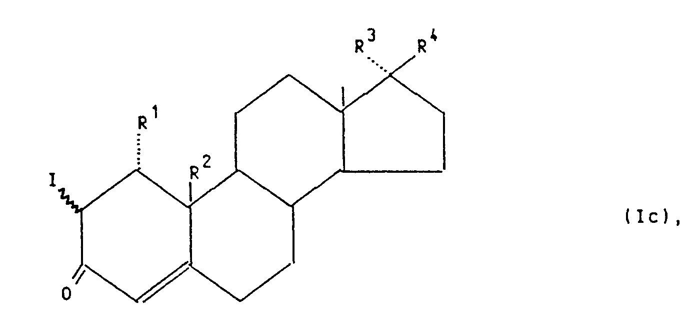 umgesetzt wird.

4. Verfahren nach Anspruch 3, dadurch gekennzeichnet, daß die Verbindung der allgemeinen Formel III mit N-Jodsuccinimid in Methanol, Ethanol, Propanol oder 2-Propanol umgesetzt wird.

5. Verfahren nach Anspruch 3, dadurch gekennzeichnet, daß die Verbindung der allgemeinen Formel III mit aus N-Chlorsuccinimid und Natriumjodid in situ generiertem N-Jodsuccinimid in Aceton oder Methanol umgesetzt wird.

6. Verfahren nach Anspruch 3, dadurch gekennzeichnet, daß mit Jodmonochlorid oder -bromid in Tetrahydrofuran oder einem anderen Ether oder in Aceton, Methylisobutylketon oder einem anderen Keton umgesetzt wird.

7. Verfahren nach Anspruch 3, dadurch gekennzeichnet, daß die Verbindung der allgemeinen Formel III mit elementarem Jod in Eisessig umgesetzt wird.

8. Verfahren nach Anspruch 3, dadurch gekennzeichnet, daß mit Jodmonochlorid, -bromid oder mit elementarem Jod in Gegenwart eines Carbonsäuresalzes oder eines Amins als Base umgesetzt wird.

9. Verfahren nach Anspruch 8, dadurch gekennzeichnet, daß mit Natriumacetat, Triethylamin, Pyridin oder áhnlichen Basen umgesetzt wird.

10. Verfahren nach Anspruch 3, dadurch gekennzeichnet, daß die Verbindung der allgemeinen Formel IV in Gegenwart einer equimolaren Menge Kupfer-II-oxid, -acetat, -halogenid, wobei Halogenid = Cl, Br, I ist, oder eines Kupfer-I-salzes wie Kupfer-I-chlorid umgesetzt wird.

11. Verfahren zur Einführung einer Δ¹-Doppelbindung in das Steroidgerüst bei gleichzeitigem Vorhandensein einer Δ⁴-Doppelbindung sowie gesättigter Carbonylgruppen, dadurch gekennzeichnet, daß eine Verbindung der allgemeinen Formel I 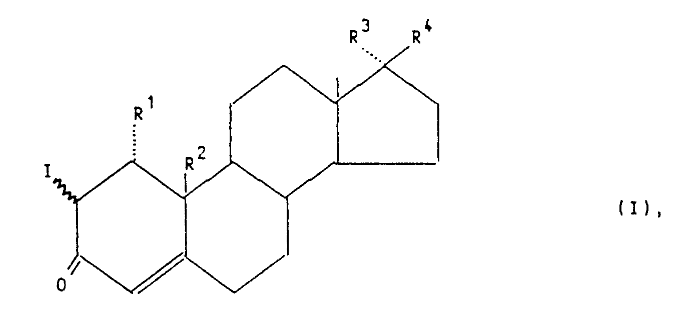 worin
R¹ für ein Wasserstoffatom oder eine geradkettige oder verzweigte Alkylgruppe mit 1 bis 4 Kohlenstoffatomen,
R² für ein Wasserstoffatom oder eine Methylgruppe,
R³ für ein Wasserstoffatom sowie
R⁴ für eine Acyloxygruppe mit 1 bis 4 Kohlenstoffatomen im Acylrest oder aber
R³ und R⁴ gemeinsam für ein Ketosauerstoffatom,
mit einer Base in einem amidischen Lösungsmittel bei erhöhter Temperatur dehydrohalogeniert wird.

12. Verfahren nach Anspruch 11, dadurch gekennzeichnet, daß mit Magnesiumoxid, Lithium-, Natrium-, Kaliumcarbonat oder dgl. dehydrohalogeniert wird.

13. Verfahren nach Anspruch 11, dadurch gekennzeichnet, daß in Dimethylformamid oder N-Methylpyrrolidon als Lösungsmittel dehydrohalogeniert wird.

14. Verfahren nach Anspruch 11, dadurch gekennzeichnet, daß bei einer Temperatur zwischen 100 und 150°C dehydrohalogeniert wird.

15. Verfahren nach Anspruch 11, dadurch gekennzeichnet, daß 2α- oder 2β-Jod-1αmethyl-androst-4-en-3,17-dion oder ein Gemisch dieser Isomere zu Atamestan (1-Methylandrosta-1,4-dien-3,17-dion) dehydrohalogeniert wird.

16. Verfahren nach Anspruch 11, dadurch gekennzeichnet, daß 2-Jod-19-nor-androst-4-en-3,17-dion zu Estron (1,3,5-Estratrien-3-ol-17-on) dehydrohalogeniert wird.

## Claims

1. Intermediate products of the general formula I' 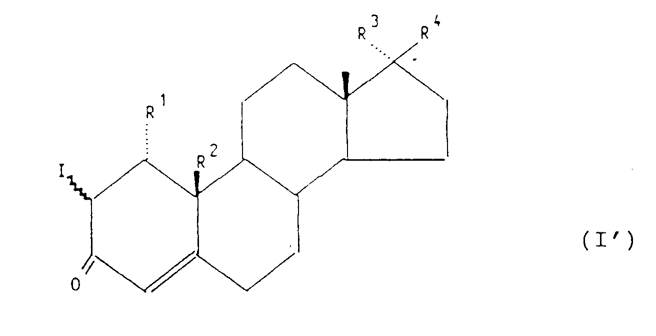 wherein
R¹ represents a hydrogen atom or a straight-chain or branched alkyl group having from 1 to 4 carbon atoms,
R² represents a hydrogen atom or a methyl group,
R³ represents a hydrogen atom and
R⁴ represents an acyloxy group having from 1 to 4 carbon atoms in the acyl radical or, alternatively,
R³ and R⁴ together represent a keto oxygen atom,
with the exception of 2-iodoandrost-4-ene-3,17-dione and 17β-acetoxy-2-iodoandrost-4-en-3-one.

2. 2β-iodo-1α-methylandrost-4-ene-3,17-dione;
2α-iodo-1α-methylandrost-4-ene-3,17-dione;
17β-acetoxy-2-iodo-1α-methylandrost-4-en-3-one;
2-iodo-19-nor-androst-4-ene-3,17-dione.

3. Process for the preparation of intermediate products of the general formula I 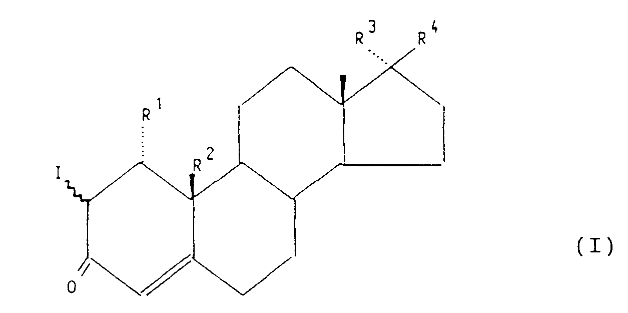 wherein R¹, R², R³ and R⁴ have the meaning given in claim 1, characterised in that either a compound of the general formula III 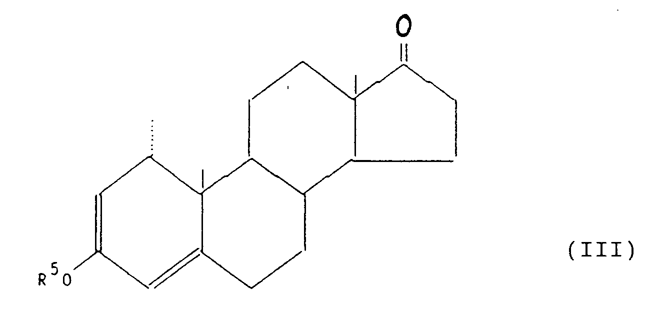 wherein
R⁵ represents an acyl or trialkylsilyl group having up to 10 carbon atoms in the group, is reacted with N-iodosuccinimide, optionally produced in situ, in a solvent to form the compound of formula Ia 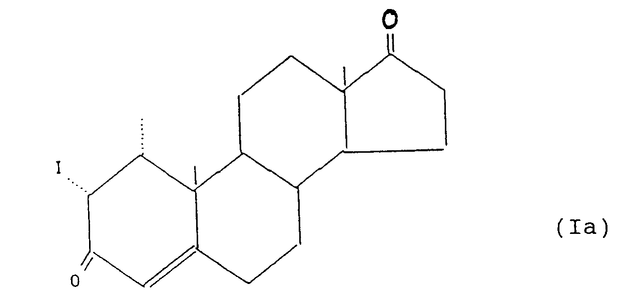 or with iodine mono-chloride or -bromide in an aprotic solvent in the presence of a base, or with elemental iodine with the addition of a base, to form the compound of formula Ib 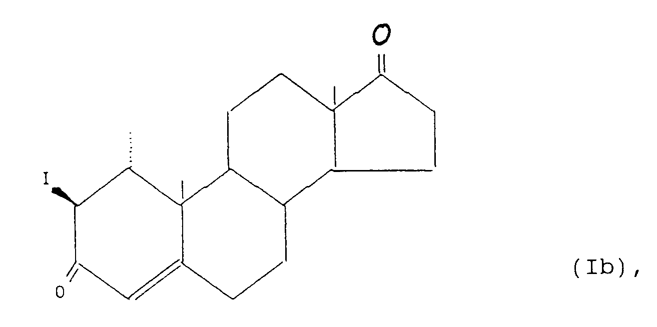 or a compound of the general formula IV 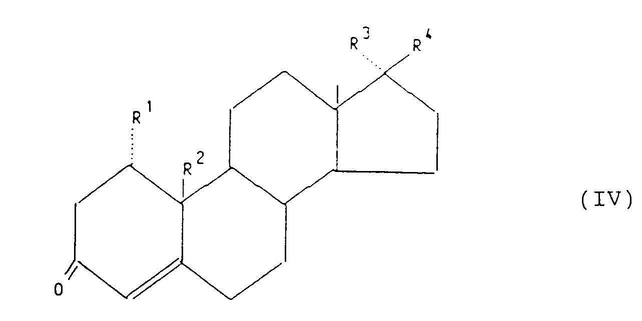 wherein R¹, R², R³ and R⁴ have the meaning given for formula I, is reacted with elemental iodine, iodine mono-chloride or -bromide, N-iodosuccinimide or another N-iodoamide or another N-iodoimide in the presence of an equimolar amount of a copper compound in acetic acid or in an inert solvent with the addition of a catalytic amount of acetic acid or another C₁-C₃carboxylic acid to form a compound of the general formula Ic

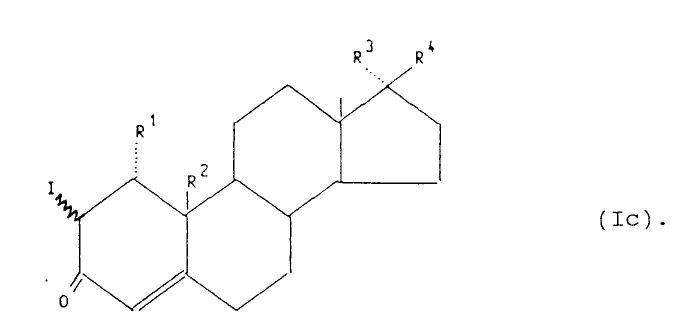

4. Process according to claim 3, characterised in that the compound of the general formula III is reacted with N-iodosuccinimide in methanol, ethanol, propanol or 2-propanol.

5. Process according to claim 3, characterised in that the compound of the general formula III is reacted in acetone or methanol with N-iodosuccinimide produced in situ from N-chlorosuccinimide and sodium iodide.

6. Process according to claim 3, characterised in that the reaction is carried out with iodine mono-chloride or -bromide in tetrahydrofuran or another ether or in acetone, methyl isobutyl ketone or another ketone.

7. Process according to claim 3, characterised in that the compound of the general formula III is reacted with elemental iodine in glacial acetic acid.

8. Process according to claim 3, characterised in that the reaction is carried out with iodine mono-chloride or -bromide or with elemental iodine in the presence of a carboxylic acid salt or of an amine as base.

9. Process according to claim 8, characterised in that the reaction is carried out with sodium acetate, triethylamine, pyridine or similar bases.

10. Process according to claim 3, characterised in that the compound of the general formula IV is reacted in the presence of an equimolar amount of copper(II) oxide, acetate or halide, halide being Cl, Br or I, or of a copper(I) salt such as copper(I) chloride.

11. Process for the introduction of a Δ¹ double bond into the steroid skeleton with the simultaneous presence of a Δ⁴ double bond and also saturated carbonyl groups, characterised in that a compound of the general formula I 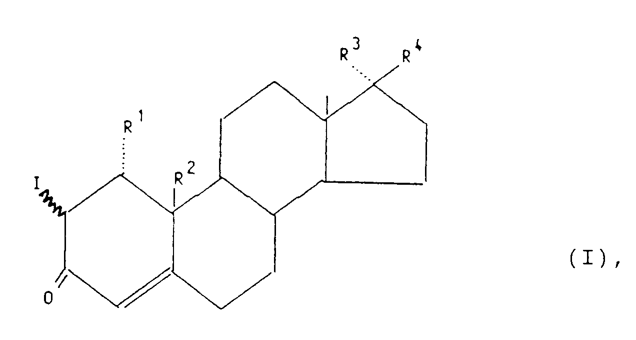 wherein
R¹ represents a hydrogen atom or a straight-chain or branched alkyl group having from 1 to 4 carbon atoms,
R² represents a hydrogen atom or a methyl group,
R³ represents a hydrogen atom and
R⁴ represents an acyloxy group having from 1 to 4 carbon atoms in the acyl radical or, alternatively,
R³ and R⁴ together represent a keto oxygen atom,
is dehydrohalogenated with a base in an amidic solvent at elevated temperature.

12. Process according to claim 11, characterised in that the dehydrohalogenation is carried out with magnesium oxide, lithium carbonate, sodium carbonate or potassium carbonate or the like.

13. Process according to claim 11, characterised in that the dehydrohalogenation is carried out in dimethylformamide or N-methylpyrrolidone as solvent.

14. Process according to claim 11, characterised in that the dehydrohalogenation is carried out at a temperature of between 100 and 150°C.

15. Process according to claim 11, characterised in that 2α- or 2β-iodo-1α-methylandrost-4-ene-3,17-dione or a mixture of those isomers is dehydrohalogenated to atamestan (1-methylandrosta-1,4-diene-3,17-dione).

16. Process according to claim 11, characterised in that 2-iodo-19-nor-androst-4-ene-3,17-dione is dehydrohalogenated to oestrone (1,3,5-oestratrien-3-ol-17-one).

## Revendications

1. Produits intermédiaires de formule générale I' 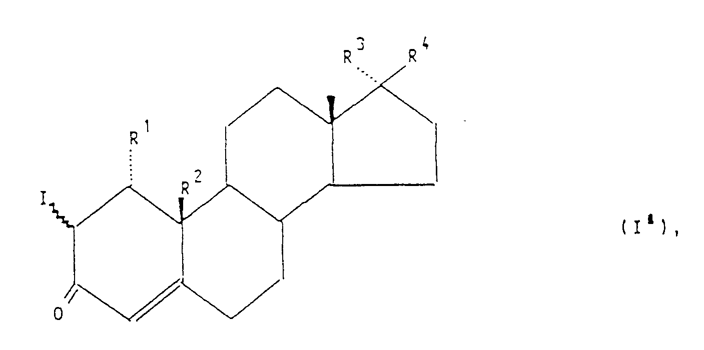 dans laquelle
R¹ désigne un atome d'hydrogène ou un alkyle linéaire ou ramifié pouvant avoir de 1 à 4 atomes de carbone,
R² un atome d'hydrogène ou le groupe méthyle,
R³ un atome d'hydrogène et
R⁴ un groupe acyloxy avec de 1 à 4 atomes de carbone dans le radical acyle, ou bien
R³ et R⁴ représentent ensemble un atome d'oxygène de groupe céto, à l'exclusion de la 2-iodo-androst-4-ène-3,17-dione et de la 17β-acétoxy-2-iodo-androst-4-ène-3-one.

2. 2β-iodo-1α-méthylandrost-4-ène-3,17-dione
2α-iodo-1α-méthylandrost-4-ène-3,17-dione
17β-acétoxy-2-iodo-1α-méthylandrost-4-ène-3-one
2-iodo-19-nor-androst-4-ène-3,17-dione.

3. Procédé de préparation de produits intermédiaires de formule générale I 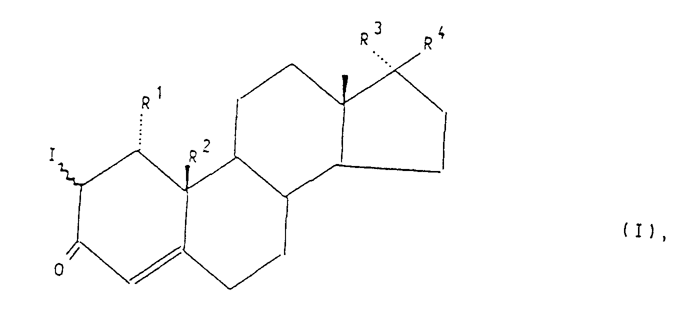 les divers symboles ayant les significations indiquées à la revendication 1, procédé caractérisé en ce que l'on fait réagir :
ou bien un composé de formule générale III 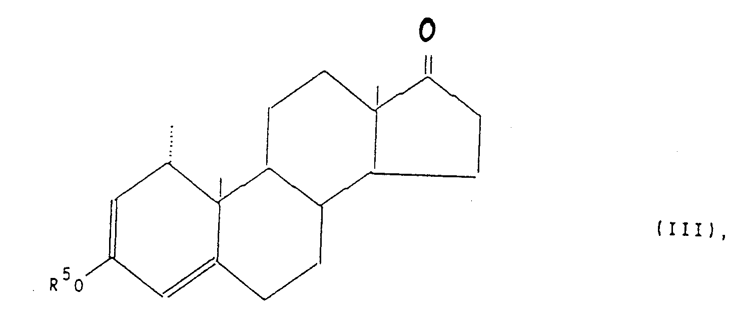 R⁵ désignant un groupe acyle ou trialkylsilyle pouvant avoir jusqu'à 10 atomes de carbone, avec, éventuellement formé insitu, du N-iodo-succinimide dans un solvant, pour former un composé de formule Ia 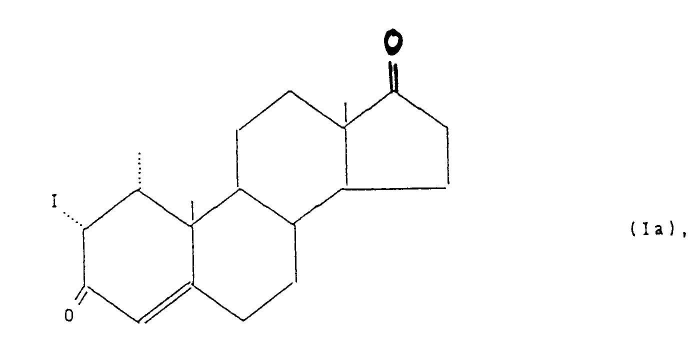 ou avec le monochlorure ou le monobromure d'iode dans un solvant aprotique en présence d'une base ou encore avec de l'iode élémentaire dans de l'acide acétique en présence d'une base, pour former un composé de formule Ib 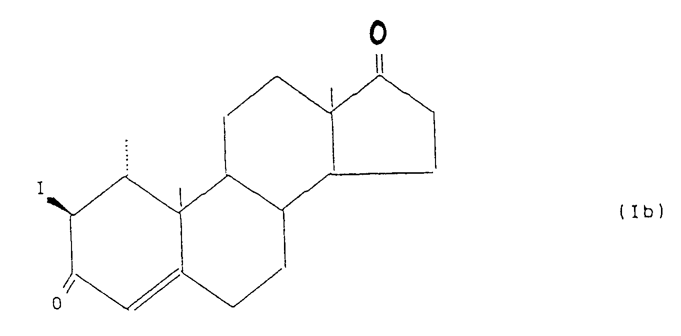 ou bien un composé de formule IV 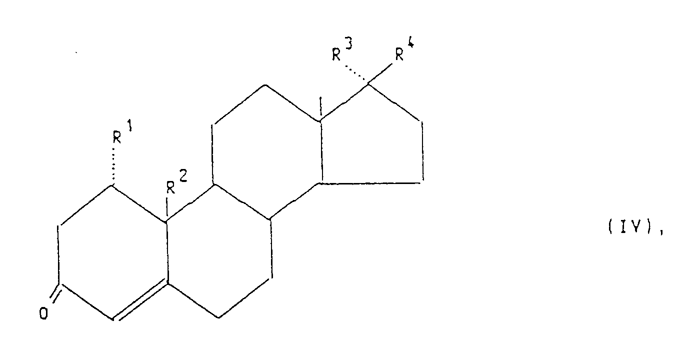 les divers symboles ayant les significations indiquées pour la formule I, avec de l'iode élémentaire, le monochlorure ou le monobromure d'iode, le N-iodo-succinimide ou un autre N-iodamide ou N-iodimide en présence d'une proportion équimolaire d'un composé de cuivre dans de l'acide acétique, ou dans un solvant inerte en présence d'une proportion catalytique d'acide acétique ou d'un autre acide carboxylique en C₁-C₃, pour former un composé de formule Ic

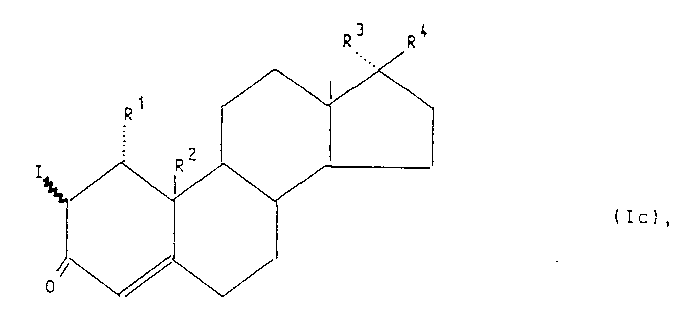

4. Procédé selon la revendication 3, caractérisé en ce que l'on fait réagir le composé de formule III avec le N-iodo-succinimide dans du méthanol, de l'éthanol, du propanol ou du 2-propanol.

5. Procédé selon la revendication 3, caractérisé en ce que l'on fait réagir le composé de formule III avec du N-iodo-succinimide formé in-situ à partir de N-chloro-succinimide et d'iodure de sodium, dans de l'acétone ou du méthanol.

6. Procédé selon la revendication 3, caractérisé en ce que l'on effectue la réaction avec du monochlorure ou du monobromure d'iode dans du tétrahydrofuranne ou un autre éther ou bien dans de l'acétone, de la méthylisobutylcétone ou une autre cétone.

7. Procédé selon la revendication 3, caractérisé en ce que l'on fait réagir le composé de formule III avec de l'iode élémentaire dans de l'acide acétique cristallisable.

8. Procédé selon la revendication 3, caractérisé en ce que l'on effectue la réaction avec le monochlorure ou le monobromure d'iode ou avec l'iode élémentaire en présence d'un sel d'acide carboxylique ou d'une amine comme base.

9. Procédé selon la revendication 8, caractérisé en ce que l'on effectue la réaction en présence d'acétate de sodium, de triéthylamine, de pyridine ou d'une autre base semblable.

10. Procédé selon la revendication 3, caractérisé en ce que l'on fait réagir le composé de formule IV en présence d'une proportion équimolaire d'oxyde, d'acétate ou d'un halogénure cuivrique (chlorure, bromure ou iodure) ou bien d'un sel cuivreux comme le chlorure cuivreux.

11. Procédé pour fixer une double liaison Δ¹ à la structure de stéroïdes en la présence d'une double liaison Δ⁴ et de groupes carbonyliques saturés, procédé caractérisé en ce que l'on soumet à une déshydrohalogénation avec une base dans un solvant amidique, à chaud, un composé de formule générale I 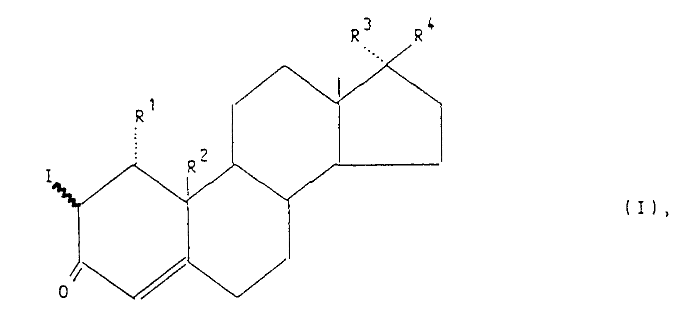 dans laquelle
R¹ représente un atome d'hydrogène ou un alkyle à chaîne linéaire ou ramifiée ayant de 1 à 4 atomes de carbone,
R² un atome d'hydrogène ou le groupe méthyle,
R³ un atome d'hydrogène et
R⁴ un groupe acyloxy avec de 1 à 4 atomes de carbone dans le radical acyle, ou bien
R³ et R⁴ représentent ensemble un atome d'oxygène cétonique.

12. Procédé selon la revendication 11, caractérisé en ce que l'on effectue la déshydrohalogénation avec de l'oxyde de magnésium, du carbonate de lithium, de sodium ou de potassium ou une autre base semblable.

13. Procédé selon la revendication 11, caractérisé en ce que l'on effectue la déshydrohalogénation dans du diméthylformamide ou de la N-méthylpyrrolidone comme solvant.

14. Procédé selon la revendication 11, caractérisé en ce que l'on effectue la déshydrohalogénation à une température comprise entre 100 et 150°C.

15. Procédé selon la revendication 11, caractérisé en ce que l'on déshydrohalogène la 2α ou 2β-iodo-1α-méthylandrost-4-ène-3,17-dione ou un mélange de ces isomères en atamestan (1-méthylandrosta-1,4-diène-3,17-dione).

16. Procédé selon la revendication 11, caractérisé en ce que l'on déshydrohalogène la 2-iodo-19-nor-androst-4-ène-3,17-dione en oestrone(1,3,5-oestratriène-3-ol-17-one).
